Europäisches Patentamt

European Patent Office

Office européen des brevets

(19)

(11) Veröffentlichungsnummer : **0 016 988**
**B1**

(12) # EUROPÄISCHE PATENTSCHRIFT

(45) Veröffentlichungstag der Patentschrift :
24.11.82

(21) Anmeldenummer : 80101103.2

(22) Anmeldetag : 05.03.80

(51) Int. Cl.³ : **A 61 K   6/10, C 08 L 83/04**

(54) Thixotrop eingestellte Silikonpasten und deren Verwendung für Zahn- und Schleimhaut-Abformungen.

(30) Priorität : 17.03.79 DE 2910560

(43) Veröffentlichungstag der Anmeldung :
15.10.80 (Patentblatt 80/21)

(45) Bekanntmachung des Hinweises auf die Patenterteilung : 24.11.82 Patentblatt 82/47

(84) Benannte Vertragsstaaten :
AT BE CH DE FR GB IT NL SE

(56) Entgegenhaltungen :
DE A 2 626 940
FR A 1 353 711
FR A 2 366 334
US A 3 082 527

Die Akte enthält technische Angaben, die nach dem Eingang der Anmeldung eingereicht wurden und die nicht in dieser Patentschrift enthalten sind.

(73) Patentinhaber : BAYER AG
Zentralbereich Patente, Marken und Lizenzen
D-5090 Leverkusen 1, Bayerwerk (DE)

(72) Erfinder : Leusner, Bernhard, Dr.
Grüner Weg 148
D-5090 Leverkusen (DE)
Erfinder : Schulz, Hans-Hermann
Am Neulandkreuz 23
D-5653 Leichlingen (DE)
Erfinder : Voigt, Reiner, Ing.Grad.
Gustav-Radbruch-Strasse 14
D-5090 Leverkusen 3 (DE)
Erfinder : Walkowiak, Michael, Dr.
Albertus-Magnus-Strasse 10
D-5090 Leverkusen 1 (DE)

# Thixotrop eingestellte Silikonpasten und deren Verwendung für Zahn- und Schleimhaut-Abformungen

Die vorliegende Erfindung betrifft thixotrop eingestellte Silikonpasten zum Herstellen genauer Abformungen von Zähnen und Schleimhäuten. Bei den erfindungsgemäßen Pasten handelt es sich um « Basismischungen » für kaltvulkanisierende Zweikomponenten-Silikonkautschuksysteme ; bei der Anwendung wird die Basismischung mit einer Katalysator/Vernetzer-Mischung, bestehend aus einem Metallsalz einer Monocarbonsäure als Katalysator und einem Kieselsäureester als Vernetzer, vermengt und innerhalb von 2-5 Minuten bei Raumtemperatur vernetzt.

In der Zahnheilkunde findet kalthärtender Silikonkautschuk als Abformmasse zur Abformung der bezahnten oder unbezahnten Kiefer bei der Herstellung von Zahnersatz zunehmende Anwendung. Je nach Indikation wird das Material in Form verschiedener konsistenter Pasten verwendet, denen vor Gebrauch ein Härter untergemischt wird. Der Übergang von pastöser streichfähiger Konsistenz in den gummielastischen Zustand findet bei Körpertemperatur innerhalb von etwa 4 bis 5 Minuten statt. Nach dieser Zeit kann der Abdruck infolge der Elastizität des Materials ohne Schwierigkeiten aus dem Munde entfernt und sodann zur Herstellung eines Arbeitsmodells z.B. mit Gips ausgegossen werden.

Der Vorteil dieser Silikonabdruckmasse gegenüber dem früher gebräuchlichen Gips liegt in der Elastizität des ausgehärteten Materials, seiner Unzerbrechlichkeit und der guten Abtrennbarkeit vom Kiefer. Auch gegenüber den heute viel gebrauchten Alginaten besitzen Siliconabdruckpasten Vorteile. Sie sind nicht wie jene empfindlich gegen Wasserverlust, der die Ursache von Schrumpfungserscheinungen sein kann, und besitzen bessere Abbildegenauigkeit. Die Maßhaltigkeit von Silikonabdrücken ist hervorragend und bleibt selbst bei langer Lagerung an der Luft gewahrt (vgl. das Buch « Chemie und Technologie der Silikone » von Walter Noll, Verlag Chemie, Weinheim, Bergstraße, 1968 Kapitel 10, 13.2).

Je nach Konsistenz der Silikon-Abformmassen werden die verschiedensten Füllstoffe verwendet : hochdisperse Kieselsäuren, Calciumsilikate, Calciumcarbonate, Bimsstein, Talkum, Calciumsulfat, Quarz- und Cristobalitmehl.

Bei den dünnfließenden Abformmassen mit Viskositäten um 10 000 mPa.s ist man auf den Einsatz von spezifisch leichten Füllstoffen angewiesen, um eine Entmischung zu vermeiden. So ist es ganz natürlich, daß solche Massen ein gewisses thixotropes Verhalten aufzeigen.

Dennoch wurde dieser Effekt nie bewußt verstärkt, um eine Paste zu erhalten, die beim Vermischen mit der Katalysator-Vernetzer-Komponente, beim Auftragen auf den Vorabdruck bzw. beim Ausdrücken aus der Spritze eine niedrige Viskosität besitzt und im Mund des Patienten infolge des Thixotropie-Effektes einen Viskositätsanstieg aufweist, der das weitere Fließen der Paste nach der Applikation verhindert.

Aus US-PS 3 082 527 sind Zahnabformmassen auf Basis von Silikonpasten bekannt, die aus einem Silikonöl (z.B. einem Dimethylpolysiloxan mit endständigen OH-Gruppen), einem Emulgator (ethoxylierter Fettalkohol) und Calciumsilikat bestehen. Ähnliches gilt für FR-A 2 366 334, worin Silikonpasten aus Polydiorganosiloxan mit endständigen OH-Gruppen, Calciumsilikat und gegebenenfalls gewissen organischen Zusatzstoffen wie z.B. Rizinusöl oder Paraffin (Vaseline) beschrieben werden.

Es zeigte sich jedoch, daß nur dann für die Praxis brauchbare Abformmassen erhalten werden, wenn man Silikonpasten verwendet, die Calciumsilikat mit einer bestimmten spezifischen Oberfläche und außerdem bestimmte ausgewählte Thixotropiermittel (nämlich hydriertes Rizinusöl oder Polyglykolether von Glyzeriden) enthalten.

Gegenstand der Erfindung sind somit thixotrope Silikon-Abformpasten, bestehend im wesentlichen aus

a) 50-90 Gewichtsprozent, bezogen auf Paste, eines Silikonöls mit endständigen Hydroxylgruppen,

b) 1-15 Gewichtsprozent, bezogen auf Paste, eines organischen Thixotropiermittels und

c) 5-50 Gewichtsprozent, bezogen auf Paste, an Calciumsilikat,

welche dadurch gekennzeichnet sind, daß das Calciumsilikat eine spezifische Oberfläche von 20-100 m²/g, vorzugsweise 35-65 m²/g (nach BET) aufweist und als Thixotropiermittel Polyglykolether von Glyzeriden oder hydriertes Rizinusöl eingesetzt werden.

Gegenstand der Erfindung ist weiterhin die Verwendung solcher Pasten mit einer Viskosität zwischen 10 000 und 100 000 mPa.s bei 23 °C (gemessen im Brookfield-Viskosimeter) für die Korrektur-Abformung sowie die Verwendung von Pasten mit einer Viskosität zwischen 30 000 und 800 000 mPa.s für die Schleimhautabformung.

Je nach Indikation kann man gegebenenfalls die Thixotropierung weiter verstärken, so daß auf die üblicherweise durchgeführte Randverformung mit z.B. knetbaren Silikon-Abformmassen (z.B. zur Darstellung von Extensionsgebieten bei der Abformung unbezahnter Unterkiefer) verzichtet werden kann.

Bei den in den erfindungsgemäßen Pasten enthaltenen Silikonölen handelt es sich vornehmlich um Hydroxylgruppen-haltige Polydiorganosiloxane. Diese können mit beliebigen einwertigen Kohlenwasserstoff- oder halogenierten Kohlenwasserstoffresten substituiert sein, z.B. mit Alkylresten mit 1-8 C-Atomen, wie Methyl, Ethyl, Propyl, Butyl, Pentyl, Hexyl, Heptyl und Octyl mit Alkenylresten mit 1-8 C-Atomen wie Vinyl, Allyl oder Butenyl, mit halogenierten Alkylresten wie Chlorpropyl oder 3,3,3-Trifluorpropyl, mit Cycloalkylresten wie Cyclobutyl, Cyclopentyl und Cyclohexyl, ferner mit aromatischen Resten wie Phenyl, Tolyl, Xylyl, und Naphthyl oder halogenaromatischen Resten wie Chlorphenyl oder Chlornaphthyl sowie mit Alkylarylresten wie Benzyl und Phenylethyl.

Die Diorganopolysiloxane können außer den Diorganosiloxy-Einheiten ($R_2SiO$) noch Triorganosiloxy-($R_3SiO_{0,5}$), Monoorganosiloxy-($RSiO_{1,5}$)- und nicht substituierte Siliciumdioxid-($SiO_2$) Einheiten enthalten.

Die Viskosität der eingesetzten Silikonöle liegt in der Regel zwischen 500 und 20 000 mPa.s, vorzugsweise um 2 000 mPa.s.

Die aus Silikonöl, organ. Thixotropiermittel und Calciumsilikat bestehende Basismischung wird mit einer an sich bekannten Katalysator-Vernetzer-Mischung vermengt und härtet zwischen 2 und 5 Minuten bei Raumtemperatur aus. Als vernetzende Substanzen können O-Kieselsäure-, Polykieselsäureester oder substituierte Silanvernetzer eingesetzt werden. Beispiele für O-Kieselsäureester sind O-Kieselsäuremethyl-, ethyl-, -n-propyl-, -isopropyl-, -chlorethyl-, -octyl-, allyl-, -hexenyl-, -cyclohexyl-, -phenyl-, -benzyl-, -chlorphenyl- und gemischt alkylierte O-Kieselsäureester wie O-Kieselsäuredimethyl-diethylester. Beispiele für die durch partielle Hydrolyse und Kondensation bzw. durch Temperung der oben aufgeführten O-Kieselsäureester entstehenden Polykieselsäureester sind Methyl-, Ethyl- und n-Propylpolysilikat.

Beispiele für Silanvernetzungsmittel sind Methyltrimethoxysilan, Methyltriethoxysilan, Ethyltrimethoxysilan, Ethyltriethoxysilan, n-Propyltrimethoxysilan, n-Propyltriethoxysilan, Phenyltrimethoxysilan, Phenyltriethoxysilan und Methyltriacetoxysilan.

Die Menge der zuzusetzenden Vernetzerverbindung beträgt in der Regel 0,1 bis 10 Gew.-Teile pro 100 Gewichtsteile Polyorganosiloxan.

Als Härtungskatalysatoren können für die erfindungsgemäßen Pasten alle Katalysatoren verwandt werden, die die Silanolkondensation, die Reaktion von Silanol mit Alkoxy- oder anderen durch Wasser hydrolysierbaren Gruppen sowie die Hydrolyse von an Silicium gebundenen Alkoxy- oder anderen Gruppen fördern.

Beispiele hierfür sind die Metallsalze organischer Monocarbonsäuren, die als Metall Blei, Zinn, Zirkon, Antimon, Eisen, Cadmium, Calcium, Barium, Mangan, Wismut oder Titan enthalten können, wie Dibutylzinndilaurat, Dibutylzinndiacetat, Zinn-II-octoat, Bleilaurat, Kobaltnaphthenat, Tetrabutyltitanat, Tetraoctyl-titanat und Tetraisopropyltitanat. Ferner Amine wie n-Hexylamin, Cyclohexylamin, Aminsalze wie Hexylaminhydrochlorid, Butylaminacetat und Guanidin-di-ethylhexoat. Carbonsäuresalze von Zinn sind als Katalysator bevorzugt. Der Zusatz an Härtungskatalysator beträgt in der Regel zwischen 0,1 und 10 %, bezogen auf das eingesetzte Organopolysiloxan.

Überraschenderweise wurde gefunden, daß die Kombination von organischem Thioxotropiermittel, vorzugsweise Glyzerinmonostearat-Polyglykoläther, mit Calciumsilikat, welches eine spez. Oberfläche von ca. 50 m²/g besitzt, zu einem besonders starken Thixotropie-Effekt führt.

So ist es in einer bestimmten Zusammensetzung möglich, daß die Paste für die Korrekturabformung strangdosierbar ist, also eine hohe Viskosität aufweist, beim Mischen mit der Vernetzer-Katalysator-Komponente und beim Applizieren eine niedrige Viskosität annimmt, so daß die Paste in enge Spalten fließen kann und anschließend wieder höher viskos wird und somit ein Abfließen vom Applikationsort verhindert.

In einer anderen Zusammensetzung der Basismischung wird durch den Thixotropie-Effekt einmal die Schleimhautoberfläche detailgenau abgeformt und zum anderen in der statischen Muskelphase das bei bisher nicht thixotrop eingestellten niedrig viskosen Abformmassen übliche Abfließen unterbunden.

Die bisher gebräuchlichen mittel- oder niedrigviskosen Silikonpasten haben den Nachteil, daß sie nach der Applikation im Munde weiter fließen und u.U. vom Applikationsort abtropfen. Hierdurch kommt es häufig durch Abfließen der Masse in den Rachenraum zur Auslösung eines Würgereizes und damit zu einer Belästigung des Patienten. Vielfach wird hierdurch die Abformung unbrauchbar.

Die erfindungsgemäßen thixotropen Massen verhindern einerseits das Abtropfen der Paste vom Applikationsort, zeichnen sich aber andererseits noch durch ein gutes Fließen unter geringem Druck aus, so daß die abzuformenden Oberflächen exakt dargestellt werden. Anhand der folgenden Beispiele soll die vorliegende Erfindung noch näher erläutert werden.

Beispiel 1

In einer Planeten-Misch- und Knetmaschine mit 1,5 l Mischbehälter werden 550 g eines endständige OH-Gruppen aufweisenden Polydimethylsiloxans mit einer Viskosität von ca. 2 000 mPa.s bei 23 °C und 456 g Calciumsilikat mit einer spez. Oberflächen von 50 ± 15 m²/g (nach BET) 30 Minuten gemischt.

In einem zweiten Mischbehälter der Planeten-Misch- und Knetmaschine werden 619 g des Polydimethylsiloxans mit einer Viskosität von ca. 2 000 mPa.s mit 25 g eines Glyzerinmonostearat-Polyglykolethers bei 70 °C verrührt, 357 g der wie oben hergestellten Silikonöl-Calciumsilikat-Mischung hinzugefügt und 15 Minuten bei 70 °C verrührt. Anschließend wird unter Kühlung mit kaltem Wasser 30 Minuten nachgemischt.

Beispiel 2

In einem Mischbehälter der Planeten-Misch- und Knetmaschine werden 490 g des Polydimethylsil-

3

## 0 016 988

oxans mit einer Viskosität von ca. 2 000 mPa.s bei 23 °C mit 30 g eines Glyzerinmonostearat-Polyglykolethers bei 70 °C verrührt, die Temperatur wird nach Zugabe von 478 g der in Beispiel 1 genannten Silikon-Calciumsilikat-Mischung 15 Minuten auf 70 °C gehalten. Anschließend wird unter Kühlung mit kaltem Wasser 30 Minuten nachgemischt.

### Beispiel 3

835 g des obigen Polydimethylsiloxans werden in einem Mischbehälter der Planeten-Misch- und Knetmaschine mit 35 g eines hydrierten Rizinusöles bei 90 °C gemischt, anschließend unter Rühren mit kaltem Wasser abgekühlt und danach 130 g eines Calciumsilikates mit einer speziellen Oberfläche von ca. 50 g/cm² (nach BET) eingerührt. Nach 30 Minuten Nachrührzeit wird die Paste über einen Homogenisator gegeben.

### Beispiele 4-6 (Anwendungsbeispiele)

300 g Tetraethoxysilan und 300 g Dibutylzinndilaurat werden in einem 1 l Kolben mit Rührer und Stickstoffanschluß bei 120 °C gerührt.

Jeweils 6 kg Basismischung aus Beispiel 1, 2 oder 3 werden mit 0,25 g der obigen Vernetzer-Katalysator-Komponente 30 Sekunden innig vermischt. Ein Teil wird nach einer Minute in eine kleine Metallkappe überführt ; man streift zu einer glatten Oberfläche ab und gibt die Metallkappe mit der Vulkanisationsmischung in einen Thermostaten mit Wasser von 37 °C. Man mißt die Härte der Vulkanisate in Shore A jeweils nach 4'30", 6', 8' und 10'.

Mit einem anderen Teil der Abmischung aus Basismischung und Vernetzer-Katalysator-Komponente prüft man das Verarbeitungsverhalten durch Ausführung des sogenannten « Digitaltests ». Man nimmt hierzu einen Teil der Abmischung zwischen Daumen und Zeigefinger und bewegt beide gegeneinander, bis die Vulkanisationsmischung « abreißt ». Die Zeit vom Beginn des Abmischens bis zum « Abreißen » ist der Digitaltest.

Die folgende Tabelle enthält Viskositätsangaben für die Basismischungen und Vulkanisationsergebnisse von deren Abmischungen.

| Basismischung aus Beispiel | 1 | 2 | 3 |
|---|---|---|---|
| Viskosität in mPa.s bei 23 °C, gemessen mit Brookfield-Viskosimeter RVT. | | | |
| Spindel Nr | 6 | 7 | 6 |
| 0,5 U/min. | 90 000 | 800 000 | 102 000 |
| 1,0 U/min. | 63 000 | 560 000 | 76 800 |
| 2,5 U/min. | 41 200 | 312 000 | 52 800 |
| 5,0 U/min. | 31 000 | 204 000 | 33 600 |
| 10,0 U/min. | 24 000 | 142 000 | 22 400 |
| 20,0 U/min. | 19 000 | 94 000 | 15 800 |
| 50,0 U/min. | 14 800 | 62 000 | 11 300 |

| Basismischung aus Beispiel | 1 | 2 | 3 |
|---|---|---|---|
| Menge Basismischung in g | 6,00 | 6,00 | 6,00 |
| Menge Vernetzer-Katalysator-Komponente in g | 0,25 | 0,25 | 0,25 |
| Digitaltest | 2'55" | 2'35" | 2'45" |

| Basismischung aus Beispiel | 1 | 2 | 3 |
|---|---|---|---|
| Shore A Härte nach 4 1/2 Min. | 17 | 21 | 16 |
| Shore A Härte nach 6 1/2 Min. | 23 | 31 | 25 |
| Shore A Härte nach 8 1/2 Min. | 29 | 34 | 30 |
| Shore A Härte nach 10 1/2 Min. | 31 | 36 | 33 |

4

## Ansprüche

1. Thixotrope Silikon-Abformpasten, bestehend im wesentlichen aus

a) 50-90 Gewichtsprozent, bezogen auf Paste, eines Silikonöls mit endständigen Hydroxylgruppen,

b) 1-15 Gewichtsprozent, bezogen auf Paste, eines organischen Thixotropiermittels und

c) 5-50 Gewichtsprozent, bezogen auf Paste, an Calciumsilikat,

dadurch gekennzeichnet, daß das Calciumsilikat eine spezifische Oberfläche von 20-100 m$^2$/g (nach BET) aufweist und als Thixotropiermittel Polyglykolether von Glyzeriden oder hydriertes Rizinusöl eingesetzt werden.

2. Pasten nach Anspruch 1, dadurch gekennzeichnet, daß als Thixotropiermittel Glyzerin-monostearat-Polyglykolether eingesetzt werden.

3. Pasten nach Anspruch 1 und 2, dadurch gekennzeichnet, daß das Calciumsilikat eine spezifische Oberfläche von 35-65 m$^2$/g aufweist.

4. Paste nach Anspruch 1 bis 3, dadurch gekennzeichnet, daß das Silikonöl ein Polydimethylsiloxan mit endständigen Hydroxylgruppen und einer Viskosität von 500-20 000 mPa.s, vorzugsweise ca. 2 000 mPa.s, bei 23 °C ist.

5. Verwendung von Pasten nach Anspruch 1 bis 4 mit einer Viskosität zwischen 10 000 und 100 000 mPa.s bei 23 °C für die Korrektur-Abformung.

6. Verwendung von Pasten nach Anspruch 1 bis 4 mit einer Viskosität zwischen 30 000 und 800 000 mPa.s bei 23 °C für die Schleimhautabformung.

## Claims

1. Thixotropic silicone impression pastes, consisting essentially of

a) 50-90 per cent by weight, based on the paste, of a silicone oil containing terminal hydroxyl groups,

b) 1-15 per cent by weight, based on the paste, of an organic thixotropic agent and

c) 5-50 per cent by weight, based on the paste, of calcium silicate,

characterised in that the calcium silicate has a specific surface area of 20-100 m$^2$/g (measured by the BET method) and polyglycol ethers of glycerides, or hydrogenated castor oil are used as thixotropic agents.

2. Pastes according to Claim 1, characterised in that glycerol monostearate polyglycol ethers are used as thixotropic agents.

3. Pastes according to Claim 1 and 2, characterised in that the calcium silicate has a specific surface area of 35-65 m$^2$/g.

4. Paste according to Claim 1 to 3, characterised in that the silicone oil is a polydimethylsiloxane which contains terminal hydroxyl groups and has a viscosity of 500-20 000 mPa.s, preferably about 2 000 mPa.s, at 23 °C.

5. Use of pastes according to Claim 1 to 4 with a viscosity between 10 000 and 100 000 mPa.s at 23 °C for correction impressions.

6. Use of pastes according to Claim 1 to 4 with a viscosity between 30 000 and 800 000 mPa.s for mucous membrane impressions.

## Revendications

1. Pâtes de moulage thixotropes à base de silicone comprenant essentiellement :

a) 50-90 % en poids (calculé sur la pâte) d'une huile de silicone comportant des groupes hydroxy terminaux,

b) 1-15 % en poids (calculé sur la pâte) d'un agent thixotrope organique, et

c) 5-50 % en poids (calculé sur la pâte) de silicate de calcium,

ces pâtes étant caractérisées en ce que le silicate de calcium a une surface spécifique de 20-100 m$^2$/g (suivant BET = Brunauer, Emmett et Teller) tandis que, comme agents thixotropes, on utilise des polyglycoléthers de glycérides ou de l'huile de ricin hydrogénée.

2. Pâtes suivant la revendication 1, caractérisées en ce que, comme agents thixotropes, on utilise des polyglycoléthers de monostéarate de glycérine.

3. Pâtes suivant les revendications 1 et 2, caractérisées en ce que le silicate de calcium a une surface spécifique de 35-65 m$^2$/g.

4. Pâtes suivant les revendications 1 à 3, caractérisées en ce que l'huile de silicone est un polydiméthylsiloxane comportant des groupes hydroxy terminaux et ayant une viscosité de 500 à 20 000 mPa.s de préférence, d'environ 2 000 mPa.s à 23 °C.

5. Utilisation de pâtes suivant les revendications 1 à 4 ayant une viscosité comprise entre 10 000 et 100 000 mPa.s à 23 °C pour le moulage de correction.

6. Utilisation de pâtes suivant les revendications 1 à 4 ayant une viscosité comprise entre 30 000 et 800 000 mPa.s pour le moulage des muqueuses.